# EUROPEAN PATENT APPLICATION

(11) **EP 1 352 958 A1**
(43) Date of publication of application: **15.10.2003**
(21) Application number: 02716318.7
(22) Date of filing: 18.01.2002
(51) Int. Cl.: C12N 15/09, C12N 1/19, C12P 7/62

(54) **GENE DISRUPTED YEAST**

(30) Priority: 19.01.2001 JP 2001011191
(71) Applicant: KANEKA CORPORATION, Osaka-shi, Osaka 530-8288 (JP)
(72) Inventor: FUKUCHI, Ken, Akashi-shi, Hyogo 673-0866 (JP); YOKOMIZO, Satoru, Kobe-shi, Hyogo 655-0872 (JP); OSAKADA, Fumio, Okayama-shi, Okayama 700-0063 (JP); MATSUMOTO, Keiji, Nishinomiya-shi, Hyogo 663-8023 (JP); TAKAGI, Masamichi, Fuchu-shi, Tokyo 183-0051 (JP); OTA, Akinori, Saitama-shi, Saitama 331-0063 (JP)
(74) Representative: HOFFMANN - EITLE
(86) International application number: JP0200326
(87) International publication number: WO02057442

(57) **Abstract**

The invention provides a yeast in which a specific gene alone has been disrupted and to which auxotrophy is impared to construct a system for producing an industrially useful substance through gene recombination by utilizing characteristics of a yeast.

In the practice of the invention, a yeast strain in which ADE1 gene has been disrupted is constructed by homologous recombination with a chromosomal DNA by using an ADE1 gene fragment encoding phosphoribosylaminoimidazole succinocarboxamide synthase (EC6.3.2.6) of *Candida maltosa.* In the practice of the invention, then, this disrupted strain is transformed by a gene expression cassette containing a biodegradable polyester synthase gene and the obtained transformant is cultured to thereby produce biodegradable polyester.

## Description

### TECHNICAL FIELD

The present invention relates to a method of disrupting a specific chromosomal DNA occurring in a yeast based on the principle of homologous recombination, and to a gene disrupted strain. The present invention also relates to the production of an industrially useful substance using said disrupted strain.

### BACKGROUND ART

Advances in gene recombination technology have made it possible to produce industrially useful substances in large amounts utilizing prokaryotes or eukaryotes. Among eukaryotes, yeasts, in particular yeasts belonging to the genus *Saccharomyces,* have been utilized for the production of fermented food products, inclusive of sake and other alcoholic drinks for a long time, and *Candida maltosa* once had been used as a microbial protein-containing food or feed. Thus, the safety of yeasts themselves has been proved.

Yeasts can grow rapidly and can be cultivated in a higher density as compared with bacteria generally. Furthermore, yeast cells can be easily separated from culture medium as compared with bacteria and, therefore, it is possible to further simplify the process for product extraction and purification. Utilizing these characteristics, yeasts have been used as hosts in the production of useful products using recombinant DNAs, and the utility thereof has been established.

Among various yeasts, yeasts belonging to the genus *Candida,* unlike those belonging to the genus *Saccharomyces,* do not form ethanol when cultivated under aerobic conditions, and their growth is not inhibited by ethanol, either. Therefore, efficient production of their cells and hence efficient substance production is possible by continuous, high-density culture thereof. Furthermore, the anascosporogenous yeast *Candida maltosa* is characterized in that it can utilize and grow on C₆ to C₄₀ straight-chain hydrocarbons, and fats and oils such as palm oil and coconut oil, each as the only carbon source. Since this characteristic is advantageous in the production of useful substances by conversion of hydrophobic chemical substances or in the use of that yeast as a field of reaction, its utilization in the production of various compounds is expected (reference: Wolf K. (ed.): Nonconventional Yeasts in Biotechnology. A Handbook, Springer-Verlag, Berlin (1996), p411-580). Further, that characteristic is also expected to be utilizable in the production of useful substances through gene recombination in *Candida maltosa* and, therefore, works have been done energetically to develop gene expression systems for such purpose (Japanese Kokai Publication Sho-62-74287, Japanese Kokai Publication Sho-62-74288, Japanese Kokai Publication Hei-02-72822). Recently, it has been disclosed that *Candida maltosa* can be utilized in producing straight-chain dicarboxylic acids through gene recombination thereof (WO 99/04014).

In cases where a yeast is used as the host for substance production through gene recombination, it is necessary to obtain a mutant having a selective marker, such as an appropriate auxotrophic character, like in the case of using *Escherichia coli* and the like. Such mutants have been obtained by random mutagenesis with a mutagen such as nitrosoguanidine or ethyl methanesulfonate. However, although this method of mutagenesis gives desired auxotrophic mutants, the possibility of mutation having been transformed into a site or sites other than the desired site cannot be denied. This poses an obstacle to development of yeast hosts and can be said to be the cause of delay in utilizing yeasts as fields of substance production as compared with *Escherichia coli* and the like.

For example, in spite of the fact that host-vector systems for *Candida maltosa* were developed fairly long ago, and a number of auxotrophic mutants thereof have been obtained by mutagenesis treatment, there was no new commercialized useful chemical substance production using recombinants of that yeast. The reason is that any auxotroph to wild strains comparable in proliferation potency and in straight-chain hydrocarbon chain utilizing capacity is not available as yet. The CHA1 strain developed by mutagenesis treatment of *Candida maltosa* has been ensured to have mutations in the ADE1 gene and HIS5 gene (Kawai S. et al., Agric. Biol. Chem., 55: 59-65 (1991)), but is poor in proliferation potency as compared with wild strains. This is considered to be due to mutation at another site or other sites than the desired sites.

Another problem with mutants obtained by random mutation is spontaneous reversion of the sites of mutation. In this case, revertants grow preferentially during culture and, therefore, the substance productivity of recombinants may decrease. Additionally, if such a revertant leaks out into the environment, the possibility of its living and growing is high, possibly posing a problem from the safety standards viewpoint. Therefore, it is not adequate to utilize yeast strains obtained by random mutagenesis as fields of substance production. Thus, it has been desired to obtain a disrupted strain with a specific auxotrophic gene alone disrupted.

As mentioned above, a number of mutants of *Candida maltosa* as resulting from mutation in the ADE1 gene, histidinol phosphate aminotransferase (HIS5 gene), orotidine 5'-phosphate decarboxylase (URA3 gene) and so forth have been obtained (reference: Wolf K. (ed.): Nonconventional Yeasts in Biotechnology. p411-580). However, any *Candida maltosa* strain with a specific gene alone disrupted and with auxotrophy has not yet been obtained. To construct a system for producing an industrially useful substance through gene recombination technology by utilizing characteristics of a yeast, such gene disrupted strains have been desired. Such gene disrupted strains not only of *Candida maltosa* but also of other yeast species have been desired.

Furthermore, it has been expected that industrially useful substances be produced in *Candida maltosa* utilizing its characteristics, namely its ability to utilize and grow on straight-chain hydrocarbons, and fats/oils such as palm oil and coconut oil, each as the only carbon source.

### SUMMARY OF THE INVENTION

The present inventors made intensive investigations in an attempt to solve the problems discussed above and, as a result, produced an ADE1 gene disrupted yeast strain by making full use of gene recombination techniques and based on the principle of homologous recombination with a chromosomal DNA by using a DNA (ADE1 gene) fragment encoding phosphoribosylaminoimidazole succinocarboxamide synthase (EC6.3.2.6) and succeeded in obtaining an adenine-requiring yeast. The proliferation potency and gene expression capacity of that gene disrupted yeast were compared with those of the ADE1 gene mutant CHA1 derived from mutagenesis treatment of *Candida maltosa,* and it was demonstrated that the gene disrupted strain is superior in both capacities to the CHA1 strain. This and other findings have now led to completion of the present invention.

Thus, the present invention relates to
a yeast in which ADE1 gene in chromosomal DNA has been disrupted by homologous recombination with an ADE1 DNA fragment. The invention also relates to
an ADE1 gene disrupted yeast transformant
which is transformed with a DNA sequence containing a homologous or heterologous gene.

Furthermore, the invention relates to
a method of producing gene expression products
which comprises cultivating a transformant of an ADE1 gene disrupted yeast and recovering a homologous or heterologous gene expression product from the culture obtained. More particularly, it provides a method of utilizing, in industrially useful substance production, a transformant obtained by transforming a homologous or heterologous gene expression system into an ADE1 gene disrupted strain of *Candida maltosa.* Here, a two-component copolymerization polyester from 3-hydroxybutyrate (hereinafter referred to as "3HB" for short) and 3-hydroxyhexanoate (hereinafter referred to as "3HH" for short) (hereinafter referred to as "P(3HB-co-3HH)" for short) is useful as a biodegradable polyester, and a polyester polymerase and enoyl-CoA hydratase are the enzymes involved in the synthesis of that polyester. The inventors succeeded in producing P(3HB-co-3HH) by transforming expression systems for these polyester polymerase gene and enoyl-CoA hydratase gene into an ADE1 gene disrupted strain of *Candida maltosa* according to the invention and thus could demonstrate the utility of the ADE1 gene disrupted *Candida maltosa.* Thus, the invention also relates to a method of producing polyesters using a transformant of an ADE1 gene disrupted strain which method comprises recovering a polyester from the culture obtained by cultivating the transformant.

### DETAILED DESCRIPTION OF THE INVENTION

The present invention is now described in detail.

The yeast to be subjected to ADE1 gene disruption by homologous recombination is not particularly restricted but use may be made of those yeasts which have been deposited with microorganism depositories (e.g. IFO, ATCC, etc.) and belong to the genera *Aciculoconidium, Ambrosiozyma, Arthroascus, Arxiozyma, Ashbya, Babjevia, Bensingtonia, Botryoascus, Botryozyma, Brettanomyces, Bullera, Bulleromyces, Candida, Citeromyces, Clavispora, Cryptococcus, Cystofilobasidium, Debaryomyces, Dekkara, Dipodascopsis, Dipodascus, Eeniella, Endomycopsella, Eremascus, Eremothecium, Erythrobasidium, Fellomyces, Filobasidium, Galactomyces, Geotrichum, Guilliermondella, Hanseniaspora, Hansenula, Hasegawaea, Holtermannia, Hormoascus, Hyphopichia, Issatchenkia, Kloeckera, Kloeckeraspora, Kluyveromyces, Kondoa, Kuraishia, Kurtzmanomyces, Leucosporidium, Lipomyces, Lodderomyces, Malassezia, Metschnikowia, Mrakia, Myxozyma, Nadsonia, Nakazawaea, Nematospora, Ogataea, Oosporidium, Pachysolen, Phachytichospora, Phaffia, Pichia, Rhodosporidium, Rhodotorula, Saccharomyces, Saccharomycodes, Saccharomycopsis, Saitoella, Sakaguchia, Saturnospora, Schizoblastosporion, Schizosaccharomyces, Schwanniomyces, Sporidiobolus, Sporobolomyces, Sporopachydermia, Stephanoascus, Sterigmatomyces,* Sterigmatosporidium, *Symbiotaphrina, Sympodiomyces, Sympodiomycopsis, Torulaspora, Trichosporiella, Trichosporon, Trigonopsis, Tsuchiyaea, Udeniomyces, Waltomyces, Wickerhamia, Wickerhamiella, Williopsis, Yamadazyma, Yarrowia, Zygoascus, Zygosaccharomyces, Zygowilliopsis,* and *Zygozyma,* among others.

Among these yeasts, the genera *Candida, Yarrowia, Kluyveromyces, Hanseniaspora, Hansenula* and the like are preferred in view of the possibility of efficient cell production and substance production by continuous high-density culture. In particular, the genus *Candida* is preferred in view of the advanced studies of the host-vector systems, hence the ready availability of such systems, and in view of the high capacity for utilizing straight-chain hydrocarbons, fats and oils, and so on.

In constructing an ADE1 gene disrupted strain and utilizing the same in accordance with the invention, *Candida maltosa* was used as an example.

The wild strain IAM 12247 of *Candida maltosa* used in the practice of the invention can be obtained from the Institute of Applied Microbiology, University of Tokyo or from the American Type Culture Collection (Manassas, VA, USA) under the accession number ATCC 28140. The *Candida maltosa* CHA1 strain, an adenine- and histidine-requiring marker mutant, can be obtained from the above collecting institution under ATCC 90625.

The ADE1 gene disrupted AC16 strain of *Candida maltosa* obtained in accordance with the invention has been deposited with the National Institute of Advanced Industrial Science and Technology International Patent Organism Depositary, Central 6, 1-1-1, Higashi, Tsukuba, Ibaraki, Japan, as of November 15, 2000 under the deposit number FERM BP-7366 under the Budapest Treaty.

Homologous recombination means recombination occurring between two DNA base sequences in a region where they have similar sequences or homologous sequences.

Gene disruption means causing mutation or inserting another DNA in the base sequence of a gene, or deleting a portion of the gene so that the gene cannot function any longer. As a result of gene disruption, the gene cannot be transcribed into mRNA, hence the structural gene is not translated, or the transcription product mRNA becomes incomplete, hence mutation or deletion occurs in the amino acid sequence of the translation product structural protein, rendering the protein incapable of performing the original function.

The ADE1 gene means a gene fragment comprising a promoter region-containing 5' untranslated region, a region coding for phosphoribosylaminoimidazole succinocarboxamide synthase (EC6.3.2.6) and a terminator region-containing 3' untranslated region. The base sequence of the ADE1 gene of *Candida maltosa* has been published in Gen Bank: D00855. The DNA sequence thereof was shown under SEQ ID NO;1.

The ADE1 DNA fragment indicates a DNA capable of causing homologous recombination with the ADE1 gene of the chromosome in the microbial cell and thereby disrupting the ADE1 gene.

The ADE1 enzyme indicates phosphoribosylaminoimidazole succinocarboxamide synthase (EC.6.3.2.6).

The term "homologous gene" means the gene occurring on the host yeast chromosome, or a partial DNA thereof. The term "heterologous gene" means a gene originally not occurring on the host yeast chromosome, or a partial DNA thereof.

A gene expression cassette is a cyclic plasmid comprising a transcription promoter DNA sequence, a DNA coding for the gene to be expressed, and a DNA containing a terminator for terminating a transcription, and includes the type capable of functioning extrachromosomally and the type to be integrated into the chromosomal DNA.

As for the gene expression product, when the substance expressed by a gene (product of gene expression) is the desired protein or enzyme, the substance itself is the gene expression product. A substance which is directly different from the product of gene expression but is produced by manifestation, in the host yeast, of catalytic activities of various enzymes and coenzymes, which are products of gene expression, is also a gene expression product.

"PHA" is an abbreviation of polyhydroxyalkanoate and stands for a biodegradable polyester resulting from copolymerization of 3-hydroxyalkanoic acids.

"ORF2" means a DNA designed based on an *Aeromonas caviae*-derived polyester polymerase-encoding gene (Japanese Kokai Publication Hei-10-108682) so as to enable expression thereof in *Candida maltosa.* The base sequence thereof was shown under SEQ ID NO:6.

"ORF3" means a DNA designed based on an *Aeromonas caviae*-derived enoyl-CoA hydratase-encoding gene (Japanese Kokai Publication Hei-10-108682) so as to enable expression thereof in *Candida maltosa.* The base sequence thereof was shown under SEQ ID NO:7.

In the following, an example of the method of constructing an ADE1 gene disrupted strain, an example of the method of producing a polyester using the disrupted strain are described.

### (1) Method of constructing an ADE1 gene disrupted strain

Any method may be used for the gene disrupted strain construction provided that a disrupted strain incapable of ADE1 enzyme expression can be obtained. While various methods of gene disruption have been reported, the gene disruption by homologous recombination is preferred in view of the fact that a specific gene alone can be disrupted (Nickoloff J. A. (ed.), Methods in Molecular Biology, 47: 291-302 (1995), Humana Press Inc., Totowa, NJ). Among the techniques of homologous recombination, the simple one-step gene disruption technique which comprises only one procedural step of transforming the gene into yeast cells is preferred in view of the fact that recombinant strains highly safe in handling can be obtained as a result of obtaining a disrupted strain with no spontaneous reversion.

The ADE1 DNA fragment generally used for one-step gene disruption is a DNA fragment derived from the gene by partial intragenic DNA elimination and rejoining of the remaining both terminal portions. Such DNA fragment can be prepared, for example, by the PCR method (polymerase chain reaction method) or by cleavage from a vector with a restriction enzyme, followed by rejoining. The homologous region length of both termini of the ADE1 DNA fragment is sufficient if it comprises at least 10 bases, preferably not less than 200 bases, more preferably not less than 300 bases. The homology of each of both termini is preferably not less than 90%, more preferably not less than 95%.

The partial DNA to be eliminated is such a portion that, upon elimination thereof, the ADE1 gene can no more manifest the enzyme activity. The length of the partial DNA is such that the ADE1 enzyme activity cannot be restored by spontaneous reversion. The chain length of such partial DNA is not particularly restricted but preferably is not less than 50 bases, more preferably not less than 100 bases.

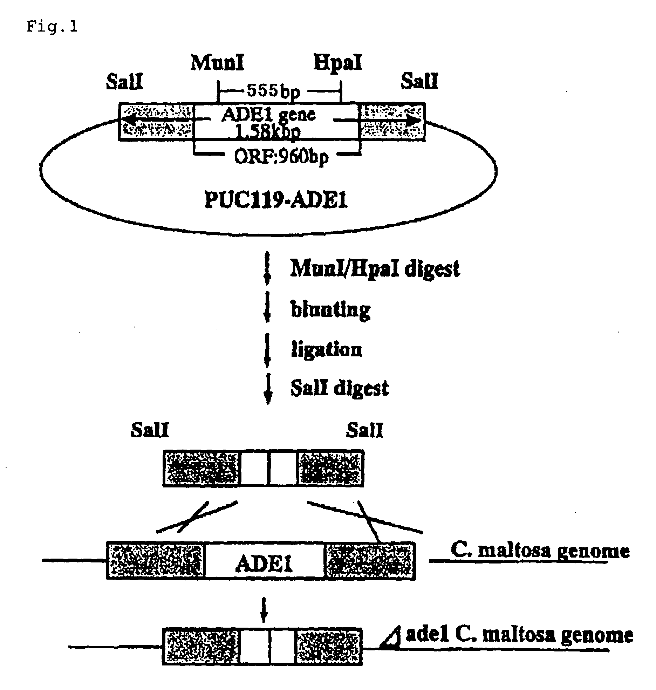

In the practice of the invention, a disrupting DNA constructed by eliminating a 555-bp ADE1 enzyme-encoding DNA fragment and ligating a 630-bp 5'-side DNA fragment and a 400 bp 3'-side DNA fragment was used (Fig. 1). The portion eliminated corresponds to about 80% of the ADE1 enzyme protein. The 5'-side and 3'-side DNA fragments both have 100% homology with the original ADE1 gene. The base sequence of the disrupting DNA fragment used in the practice of the invention was shown under SEQ ID NO:2.

The DNA fragment used in the practice of the invention was prepared using pUC119-ADE1 (Kawai S., et al., Agric. Biol. Chem., 55: 59-65 (1991)). Thus, *Escherichia coli* transformed with the plasmid pUC119-ADE1 was cultivated, and the plasmid is prepared therefrom. A portion of the structural gene (gene coding for the ADE1 enzyme protein) of the ADE1 gene was cleaved out from this plasmid with an appropriate restriction enzyme, and the vector side is recovered and again ligated using a ligase. A vector containing a disrupting DNA is thus prepared.

The disrupting DNA-containing vector is transformed into an adequate strain of *Escherichia coli,* for example JM 109 or DH 5α, the *E. coli* strain is mass-cultured, and the high-purity plasmid is mass-prepared by cesium chloride. ultracentrifugation (Sambrook et al. (eds.), Molecular cloning: A Laboratory Manual, Second Edition, 1.42-1.47, Cold Spring Harbor Laboratory Press (1989)). Although this vector as it is may be used directly for gene disruption, it is desirable for one-step disruption that an ADE1 region-containing homologous portion be cleaved from the purified vector with an appropriate restriction enzyme for use as a disrupting DNA. In the practice of the present invention, the vector was cleaved with the restriction enzyme *Sal*I, and the DNA fragment was transformed, without purification, into a yeast, whereby the ADE1 gene could be disrupted as a result of homologous recombination.

While the protoplast method, lithium acetate method (Takagi M., et al., J. Bacteriol, 167; 551-5 (1986)), and electric pulse method (Kasuske A., et al., Yeast, 8: 691-697 (1992) are known for the transformation of *Candida maltosa,* the electric pulse method was employed in the practice of the invention. A commercial apparatus can be used for electric pulse generation. In the practice of the invention, ELECTRO CELL MANIPULATOR 600 produced by BTX (San Diego, CA, USA) was used. The cuvette used was BM 6200 (2 mm gap blue cap) produced by BIO MEDICAL CORPORATION CO., LTD (Tokyo, Japan). After electric pulse application, the yeast is cultured on an appropriate medium, and the cultured cells obtained are screened for the desired ADE1 disrupted strain.

Among various methods developed for transformant recovery, the so-called nystatin concentration method (Snow R., Nature, 211: 206-207 (1966)) can be utilized. This method is a method originally developed for efficiently selecting mutants obtained by random mutation but is considered to be applicable to gene disrupted strains as well. For example, in a preferred embodiment of the present invention, the cells cultured after electric pulse application are transferred to a minimal medium, for instance, and cultured. The cells are washed, cultured on a nitrogen source-free minimal medium and, then, cultured on a nitrogen source-containing minimal medium for a short period of time. Nystatin is directly added to this culture medium, and aerobic culture is performed at 30°C for 1 hour, whereby wild strains can be killed preferentially. An appropriate agar medium plate is smeared with the cell suspension, and culture is performed at 30°C for about 2 days, whereby red colonies are obtained.

From among the red colonies obtained, gene disrupted ones can be confirmed with ease by the PCR method or the genomic southern hybridization method (Sambrook et al. (eds.), Molecular cloning: A Laboratory Manual, Second Edition, 9.31-9.57, Cold Spring Harbor Laboratory Press (1989)).

In the case of PCR, when both ends of the ADE1 gene are used as primers, a normal size DNA band is detected for the wild strain upon agarose gel electrophoresis, while a band shorter by the deleted sequence is detected for the disrupted strain. In the practice of the invention, an about 1 kbp DNA band was detected. In the case of gene substitution or integration in chromosomal DNA, such as in gene disruption, the possibility should be taken into consideration that the gene may be inserted into a site or sites other than the intended one, for example an unknown, highly homologous site or sites. In that case, the PCR method may sometimes fail to provide confirmation. The PCR method is desirably used as means for selecting candidates when a large number of disrupted strains are obtained.

For identifying a disrupted strain or the like, genomic southern analysis is essential. By carrying out this method, it is possible to demonstrate with ease and for certain that gene recombination has occurred at the intended site alone. The chromosomal DNA of the disrupted strain and that of the wild strain as a control are prepared and, after cleavage with an appropriate restriction enzyme or enzymes, agarose gel electrophoresis is carried out. In the practice of the present invention, identification was attempted by three methods of cleavage using two restriction enzymes. The DNAs were transferred from the agarose gel onto a nylon membrane, and the 264-bp DNA fragment resulting from cleavage of the ADE1 gene with the restriction enzymes *Hpa*I and *Nde*I, which is considered as remaining on the genome of the disrupted strain, was enzyme-labeled using the GeneImage labeling/detection kit (product of Amersham) and following the procedure described in the manual attached thereto and the labeling product was used as a probe for detection by southern hybridization (SEQ ID NO:5). After hybridization, the membrane was washed, and DNA band detection was performed using the fluorescence-emitting reagent according to the manual attached. The band detected was compared with that of the wild strain (IAM 12247). As a result, two strains were confirmed, by three cleavage methods, that an about 550 bp portion within the ADE1 gene had been shortened.

The ADE1 gene disrupted strains obtained cannot synthesize the ADE1 enzyme, hence cannot synthesize adenine, which is the precursor of ATP. Therefore, they cannot live in adenine-free media or in an adenine-free environment.

One of the ADE1 gene disrupted strains was named as *Candida maltosa* AC16.

### (2) Heterologous gene expression using the ADE1 gene disrupted strain: A method of polyester production

A homologous gene or heterologous gene can be expressed using the ADE1 disrupted gene obtained in accordance with the invention. Since yeasts can secrete sugar chain-added proteins into medium, unlike *Escherichia* *coli,* which cannot do so, it is possible to produce such proteins using the ADE1 disrupted strain.

The homologous gene capable of transformation is not particularly restricted but, as an example of industrially useful product production, there may be mentioned dicarboxylic acid production by transformation of the *Candida maltosa*-derived P450 enzyme gene into the same yeast strain, as disclosed in WO 99/04014. The heterologous gene is not particularly restricted, either but, for example, there may be mentioned the transformation of the lipase gene, amylase gene or the like for the production of such protein.

It is well known that, in some yeasts of the genus *Candida,* such as *Candida maltosa* used in the practice of the present invention, a codon is translated in a manner different from other organisms in the step of translation from mRNA into proteins. In *Candida maltosa,* the leucine codon CTG is translated into serine (Ohama T., et al., Nucleic Acid Res., 21: 4039-4045 (1993)) and, therefore, the *Escherichia coli*-derived lacZ gene is not translated into active β-galactosidase (Sugiyama H., et al., Yeast, 11: 43-52 (1995)). Thus, when the expression of a heterologous gene is intended, it is not always guaranteed that the gene be translated into a functional protein in *Candida maltosa.* Therefore, when a heterologous gene is to be expressed in *Candida maltosa* as the host, it is in principle necessary to convert the leucine codon alone. For more efficient expression, however, another or other amino acid codons may also be modified according to the codon usage in *Candida maltosa.* Codon conversion may be carried out referring, for example, to Mauersberger S., et al. (authors), *Candida maltosa,* in Wolf K. (ed.), Nonconventional Yeasts in Biotechnology. p524-527.

The genes to be involved in polyester synthesis may be any genes involved in the synthesis of a polyester of the general formula given below. The general formula given below represents a structural formula of the polyester P(3HB-co-3HH), and X and Y each represents an integer and respectively indicate the numbers of 3HB and 3HH units involved in polymerization.

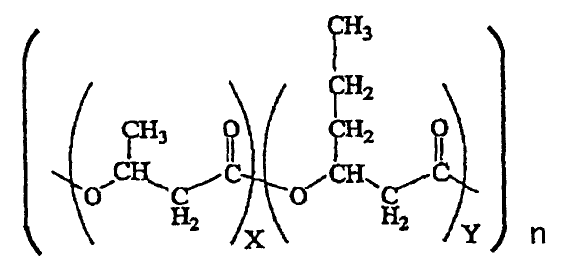

For example, the polyester polymerase gene fragment described in Japanese Kokai Publication Hei-10-108682 may be used. In addition to this polyester polymerase gene, any other gene involved in polyester synthesis may further be transformed. As such gene, there may be mentioned, for example, the (R) form-specific enoyl-CoA hydratase gene for converting enoyl-CoA, an intermediate in the β-oxidation pathway, to (R)-3-hydroxyacyl-CoA (Fukui T., et al., FEMS Microbiology Letters, 170: 69-75 (1999); Japanese Kokai Publication Hei-10-108682) and the β-ketothiolase NADH-dependent hydratase gene for synthesizing 3-hydroxybutyryl-CoA by dimerization of acetyl-CoA (Peoples O. P., et al., J. Biol. Chem., 264: 15298-15303 (1989)). These genes may contain one or more mutations, such as deletion, substitution and insertion, in the base sequence thereof as long as the expression products have substantial enzyme activity. In the case of heterologous genes, however, there is no guarantee that they are always translated into proteins capable of functioning in *Candida maltosa,* as mentioned above, hence amino acid codon modification is necessary.

In the practice of the invention, the *Aeromonas* *caviae*-derived polyester polymerase and enoyl-CoA hydratase (Japanese Kokai Publication Hei-10-108682; Fukui T., et al., FEMS Microbiology Letters, 170: 69-75 (1999)) were used. However, since the possibility that these enzyme genes may not be translated normally, hence no enzyme activities may be manifested, is high, some amino acid codons for both enzymes were modified into those optimal in *Candida maltosa,* and both the DNAs were totally synthesized. The sequences of the totally synthesized *Aeromonas caviae*-derived polyester polymerase and enoyl-CoA hydratase DNAs are shown under SEQ ID NO:6 and SEQ ID NO:7 of the sequence listing, respectively. However, neither of the base sequences shown under these sequence identifier numbers has any limitative meaning. Any other base sequences may also be used if the amino acid sequences of those enzymes are expressed thereby in *Candida maltosa.*

The gene expression cassette to be used in that yeast is constructed by ligating such DNA sequences as a promoter and 5' upstream activating sequence (UAS) to the genes in question on the 5' upstream side and ligating such DNA sequences as a poly(A) addition signal and terminator to the genes on the 3' downstream side. These DNA sequences may be any sequences if they function in the yeast in question.

The promoter includes constitutive expression type ones and inducible type ones. While either type of promoter may be used, the promoter derived from the same yeast *Candida maltosa* as the host is desirable. In an example, the *Candida maltosa* ALK1 promoter and ALK1 terminator (Gen Bank D00481) (Takagi M., et al., Agric. Biol. Chem., 5: 2217-2226 (1989)) can be utilized.

An example of the gene expression cassette to be used for transformation purposes in the practice of the present invention can be constructed as follows. The vector to be used for the construction may be any one if it is a plasmid capable of autonomously replicating in *Escherichia coli.* It may also have a region capable of autonomously replicating in the yeast. It is known that a vector capable of autonomously replicating in a yeast is retained in the yeast cells. It is also possible to integrate the gene expression cassette into the chromosome. As an example, pUTU1 capable of autonomously replicating in *Candida maltosa* can be used (M. Ohkuma, et al., J. Biol. Chem., vol. 273, 3948-3953 (1998)).

In the practice of the present invention, the *Candida maltosa* Alk1 gene promoter ALK1p (SEQ ID NO:8) was ligated to each of ORF2 and ORF3 on the 5' upstream side thereof, and the terminator ALK1t (SEQ ID NO:9) was ligated thereto on the 3' downstream side thereof.

Restriction enzyme sites for ligating the promoter and terminator to the structural gene can be prepared by utilizing the PCR method. Primer sequences suited for use in PCR are shown under SEQ ID NO:10 to SEQ ID NO:13. The PCR may be carried out under any conditions as long as the desired gene fragments can be amplified.

As for the promoter portion, ALK1p having a 5'-terminal *Sal*I site and a 3'-terminal *Nde*I site can be constructed using the template shown under SEQ ID NO:8 and the primers shown under SEQ ID NO:10 and SEQ ID NO:11. As for the terminator portion, ALK1t having a 5'-terminal *Hind*III site and a 3'-terminal *Eco*RV site can be constructed using the template shown under SEQ ID NO:9 and the primers shown under SEQ ID NO:12 and SEQ ID NO:13. Usable as the vector is the vector pUTA1 derived from pUTU1 and the *Candida maltosa* Ade1 gene by modifying the marker gene from uracil into adenine. When ALK1p is ligated to pUCNT (described in WO 94/03613) at the *Pvu*II-*Nde*I site thereof, and ALK1t to pUCNT at the *Hind*III-*Ssp*I site, pUAL1 can be constructed. Then, ORF2 is ligated to pUAL1 at the *Nde*I*-Pst*I site thereof, whereby the plasmid pUAL-ORF2 can be constructed. Further, ORF3 is ligated to pUCNT-ALK1t, constructed in the course of the construction of pUAL1, at the *Nde*I-*Hind*III site thereof, followed by further ligating of ALK1p, whereby pUAL-ORF3 can be constructed.

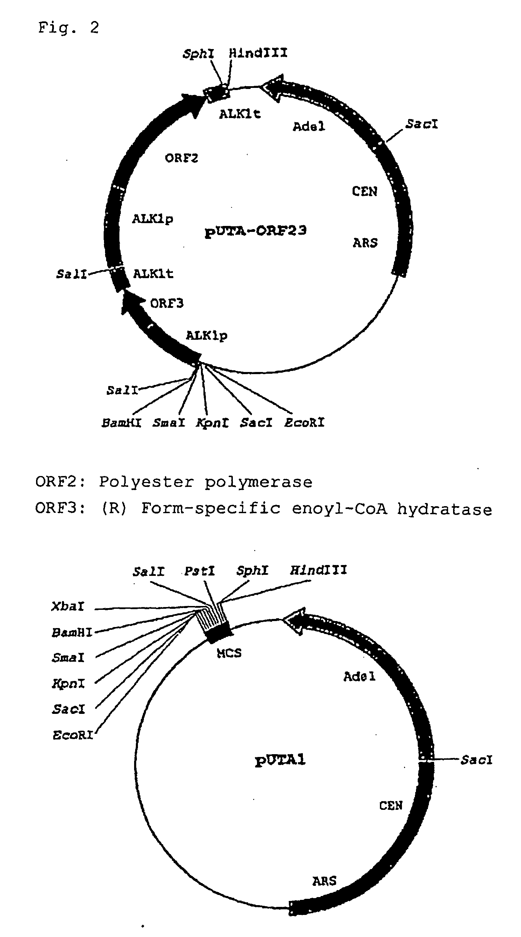

Then, ORF2, together with the upstream promoter and downstream terminator, is cleaved from the plasmid pUAL-ORF2 using *Eco*T22I and ligated to pUTA1 at the *Pst*I site thereof. In this way, pUTA-ORF2 can be constructed. Further, ORF3, together with the upstream promoter and downstream terminator, is cleaved from pUAL-ORF3 using *Sal*I and ligated to pUTA-ORF2 at the *Sal*I site thereof, whereby the plasmid pUTA-ORF23 (Fig. 2, above) can be constructed. In the above manner, a gene expression cassette for polyester production in *Candida maltosa* can be constructed.

The yeast strain of the invention is transformed with the gene expression cassette using the transformation method mentioned above, whereby the *Candida maltosa* AC16 (ORF23) strain having pUTA-ORF23 can be produced.

The polyester production by cultivating the yeast transformed with the gene expression cassette participating in the polyester synthesis can be made in the following manner. The carbon source to be used in the culture may be any of those utilizable by the yeast. When the promoter expression is of inducible type, an inducer may be timely added. The inducer may server as the main carbon source in certain instances. As for the other nutrient sources than the carbon source, media further containing a nitrogen source, an inorganic salt, and other organic nutrient sources can be used. The culture temperature may be at any level at which the yeast can grow. A temperature of 20°C to 40°C is preferred, however. The culture time is not particularly restricted but may be about 1 to 7 days. Thereafter, the polyester may be recovered from the cells or culture obtained.

The carbon source includes carbohydrates, fats and oils, fatty acids, and so forth. The carbohydrates include, among others, glucose, sucrose, glycerol, n-alkanes, and so on, and the fats and oils include, for example, rapeseed oil, coconut oil, palm oil, palm kernel oil, etc. The fatty acids include saturated and unsaturated fatty acids such as hexanoic acid, octanoic acid, decanoic acid, lauric acid, oleic acid, palmitic acid, linolic acid, linolenic acid, and myristic acid, and fatty acid derivatives such as esters and salts of such fatty acids. In an example, *Candida maltosa* can be cultured using fats or oils as the carbon source. In the case of fats and oils incapable of being utilized or capable of being utilized only inefficiently, the utilizing capacity can be improved by addition of lipase to the medium. It is also possible to provide the yeast with an ability to utilize fats and oils by transformation with a lipase gene.

As the nitrogen source, there may be mentioned ammonia, ammonium salts such as ammonium chloride, ammonium sulfate and ammonium phosphate and, further, peptone, meat extracts, yeast extracts, and so on.

The inorganic salt includes, among others, potassium dihydrogenphosphate, dipotassium hydrogenphosphate, magnesium phosphate, magnesium sulfate, sodium chloride, etc.

As other organic nutrient sources, there may be mentioned, for example, amino acids, for example glycine, alanine, serine, threonine, proline, etc., and vitamins, for example vitamin B1, vitamin B12, biotin, nicotinamide, pantothenic acid, vitamin C. etc.

As for the inducer, there may be mentioned, for example, enzymes (Alk1, Alk2, Alk3, Alk4, Alk5, etc.) (Ohkuma M., et al., J. Biol. Chem., 273: 3948-3953 (1998)) involved in fatty acid metabolism and inducible by fats and oils or by decomposition products thereof, namely fatty acids.

As regards the polyester recovery from yeast cells, a number of methods have been reported. In the practice of the present invention, such a method as mentioned below, for instance, can be used. After completion of the culture, cells are separated and recovered by subjecting the culture medium to a centrifuge or the like, and the cells are washed with distilled water and methanol, for instance, and then dried. The polyester is extracted from the dried cells with an organic solvent such as chloroform. For removing cell components, the polyester-containing organic solvent solution is subjected to filtration or other treatment, and a poor solvent, such as methanol or hexane, is added to the filtrate to cause precipitation of the polyester. The supernatant is removed by filtration or centrifugation, and the thus-collected polyester precipitate is dried. The polyester can thus be recovered.

The polyester obtained can be analyzed, for example, by gas chromatography, nuclear magnetic resonance spectrometry, etc.

### BRIEF DESCRIPTION OF THE DRAWINGS

Fig. 1 schematically illustrates pUC119-ADE1 as well as a method of constructing a DNA for ADE1 disruption.
Fig. 2 illustrates an *Aeromonas caviae*-derived polyester polymerase and enoyl-CoA hydratase expression cassette, pUTA-ORF23 (Fig. 2, upper) as well as a control cassette, pUTA1, free of both enzyme expression genes (Fig. 2, lower).
Fig. 3 shows the results of primary screening for ADE1 gene disrupted strains by PCR. C16 and C17 were considered to be disrupted strains.
Fig. 4 shows the confirmation of ADE1 gene disruption by genomic southern hybridization. In C16 and C17, the gene was found to have been disrupted normally.
Fig. 5 shows a restriction enzyme map of the vicinity of the disrupted ADE1 gene in the ADE1 gene disrupted *Candida maltosa* chromosome as revealed by southern hybridization.
Fig. 6 shows the growth curve of the ADE1 gene disrupted strain AC16 in YPD medium.
Fig. 7 shows the growth curves of the ADE1 gene disrupted strain AC16 as obtained by using palm oil, coconut oil, and tetradecane as carbon sources, respectively.
Fig. 8 shows a 400 MHz proton NMR chart of polyester P(3HB-co-3HH) produced in the AC16 (ORF23) strain. The assignment of each proton of the polyester is indicated by a number.

### BEST MODES FOR CARRYING OUT THE INVENTION

The following examples illustrate the present invention more specifically. These examples are, however, by no means limitative of the scope of the invention.

The reagents used in yeast culture were products commercially available from Wako Pure Chemical Industries, unless otherwise specified.

### (Medium composition)

LB medium: 10 g/L Tryptone, 5 g/L yeast extract, 5 g/L sodium chloride. In the case of LB plates, agar is added to 16 g/L.

YPD medium: 10 g/L Yeast extract, 20 g/L polypeptone, 20 g/L glucose. In the case of YPD plates, agar is added to 20 g/L. In the case of adenine-containing YPD medium, adenine is added 0.1 g/L.

YM medium: 3 g/L Yeast extract, 3 g/L malt extract, 5 g/L Bacto-peptone, 10 g/L glucose.

SD medium: 6.7 g/L Amino acid-free yeast nitrogen base (YNB), 20 g/L glucose. In the case of adenine-containing medium, 24 mg/L of adenine is added. In the case of SD plates, agar is added to 20 g/L.

M medium: 0.5 g/L Magnesium sulfate, 0.1 g/L sodium chloride, 0.4 mg/L thiamine, 0.4 mg/L pyridoxine, 0.4 mg/L calcium pantothenate, 2 mg/L inositol, 0.002 mg/L biotin, 0.05 mg/L iron chloride, 0.07 mg/L zinc sulfate, 0.01 mg/L boric acid, 0.01 mg copper sulfate, 0.01 mg potassium iodide, 87.5 mg/L potassium dihydrogenphosphate, 12.5 mg/L dipotassium hydrogen phosphate, 0.1 g/L calcium chloride, 20 g/L glucose. In the case of ammonium sulfate-containing M medium, 1 g/L ammonium sulfate is added. In the case of ammonium sulfate- and adenine-containing M medium, 1 g/L ammonium sulfate and 24 mg/L adenine are added to M medium.

Polyester production medium: 6.7 g/L YNB, 10 g/L casamino acids, supplemented with 20 g/L n-alkane or fat/oil as a carbon source.

Liquid culture of yeast cells was carried out using a 50-ml test tube, a 500-ml Sakaguchi flask, or a 2-L Sakaguchi flask. Shake culture was performed at 300 rpm in the case of a 50-ml test tube, at 100 to 110 rpm in the case of a 500-ml Sakaguchi flask, or at 90 to 100 rpm in the case of a 2-L Sakaguchi flask. The culture temperature was 30°C both in liquid culture and in plate culture.

### (Restriction enzyme treatment)

The restriction enzyme treatment was carried out under the reaction conditions recommended by the manufacturer or following the method described in Sambrook et al. (eds.): Molecular cloning: A Laboratory Manual, Second Edition, Cold Spring Harbor Laboratory Press (1989).

In the practice of the invention, a number of commercial kits were used and, unless otherwise specified, the procedures followed were as described in the manuals attached.

### (Example 1) Construction of a disrupting DNA

The ADE1 gene-containing vector pUC119-ADE1 (4.64 kbp) for use in *Escherichia coli* (Kawai S., et al., Agric. Biol. Chem., 55: 59-65 (1991)) was cleaved with *Mun*I and *Hpa*I*,* followed by blunting treatment using the TAKARA Blunting Kit (product of Takara Shuzo). The DNAs after blunting treatment were electrophoresed on a 1% agarose gel, and the fragment comprising the vector plus both sides of ADE1 (4.19 kbp) and the *Mun*I/*Hpa*I cleavage fragment (about 550 bp) were separated from each other. The vector plus both sides of ADE1 fragment was recovered from the agarose gel using EASYTRAP (product of Takara Shuzo), and the both sides of ADE1 were ligated using the TAKARA Ligation Kit Ver2 (product of Takara Shuzo). The reaction mixture was added to 100 µl of *Escherichia coli* competent cells DH5α to a solution ratio of not more than 5%, whereby the *Escherichia coli* strain was transformed. An adequate amount of LB medium was added and, after 1 hour of shake culture, an LB plate was smeared with the cell suspension and cultured overnight at 37°C. Several of the colonies that had been formed were picked up and cultured on LB medium in the conventional manner, and the plasmid DNA was extracted from the cultured cells. The plasmid was cleaved with the restriction enzyme *Sal*I, and the appearance of a 1.03 kbp band was confirmed by agarose gel electrophoresis. Thus, the desired vector for disruption was obtained. This transformant *Escherichia coli* strain was mass-cultured on LB medium, and the vector was mass-prepared by the cesium chloride ultracentrifugation method according to Molecular cloning: A Laboratory Manual, Second Edition 1.42-1.47, Cold Spring Harbor Laboratory Press (1989). The ADE1 DNA for disruption was cleaved using *Sal*I for one-step disruption. The whole amount of the DNA was made into a 5 mg/ml aqueous solution, and the cleaved DNA was used for gene disruption without purification.

### (Example 2) Gene disruption experiment

The *Candida maltosa* strain IAM 12247 was inoculated into 10 ml of YM medium, and precultured overnight. Then, 1 ml of the preculture medium was inoculated in 100 ml of YM medium (500-ml Sakaguchi flask) and, after 6 hours of culture, cells were collected by centrifugation. The cells were suspended in 500 µl of 1 M sorbitol, 100 µg of the disrupting DNA was added, and the mixture was gently stirred.

The gene transformation was carried out by the electric pulse method. The gene transformation apparatus used was BTX's ELECTRO CELL MANIPULATOR 600. The cuvette used was BM 6200 produced by BIO MEDICAL CORPORATION CO., LTD. A 100-µl portion of the competent cells/DNA suspension prepared was taken and poured into each cuvette, and the cuvette was set on the pulse apparatus. Then, electric pulses were applied under the following conditions: electrostatic capacity 40 µF, resistance value 246 ohm, voltage 1.9 kV. After pulse application, 1 ml of 1 M sorbitol was added to each cuvette and, after gentle mixing, the mixture was allowed to stand at room temperature for 1 hour. Then, the mixture was transferred to 100 ml of YM medium and cultured overnight.

### (Example 3) Screening for disrupted strains

Then, the cells cultured on YM medium were inoculated into 50 ml of ammonium sulfate-free M medium (500-ml Sakaguchi flask) and cultured overnight. Then, the cells were suspended in 100 ml of ammonium sulfate-containing M medium and cultured at 30°C for 6 hours. To this culture medium was added a 3 mg/ml solution of nystatin (product of Sigma) in ethanol to a final concentration of 10 µg/ml, and the mixture was cultured for 1 hour. The nystatin-treated cells were washed twice with sterilized water and again suspended in 50 ml of sterilized water. YPD plates were smeared with this cell suspension. The plates were cultured at 30°C for 2 days, whereby 24 red colonies were obtained. It was confirmed that none of these strains could grow on SD medium.

### (Example 4) Analysis by PCR

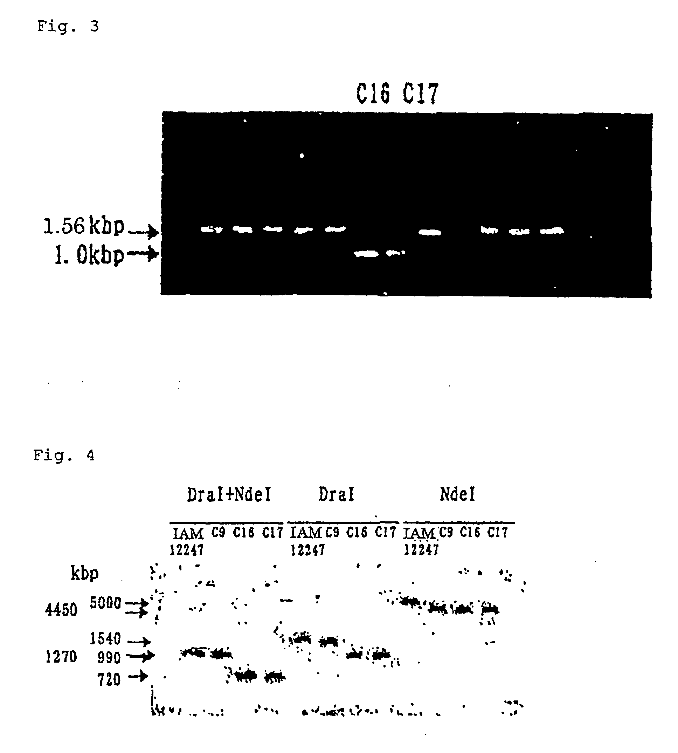

Primary screening for disrupted strains from among a large number of red colonies was carried out using the PCR method. The red colonies obtained were cultured on YPD medium. The chromosomal DNA was extracted from each culture using the chromosome extraction kit Gentle-Kun (product of Takara Shuzo). With this chromosomal DNA, PCR was carried out using both the ADE1 gene terminal primers (SEQ ID NO:3 and SEQ ID NO:4). The PCR was performed using the Perkin Elmer Amplitaq kit. The gene amplification was effected using Biometra's TRIO-Thermoblock^{TM}. Thirty cycles each comprising 1 minute at 94°C, 1 minute at 50°C and 3 minutes at 70°C were carried out. With the wild strain, the 1.56 kbp DNA is amplified and, with the gene disrupted strain, the shortened 1.0 kbp DNA is amplified. The PCR reaction mixture was subjected to 1% agarose gel electrophoresis for detecting the 1.0 kbp DNA fragment. As a result, in 3 strains out of the 24 strains, the ADE1 gene was found to have been shortened, hence considered to have been disrupted. The PCR patterns for two strains (C16, C17) out of the three strains were shown in Fig. 3.

### (Example 5) Analysis by genomic southern hybridization

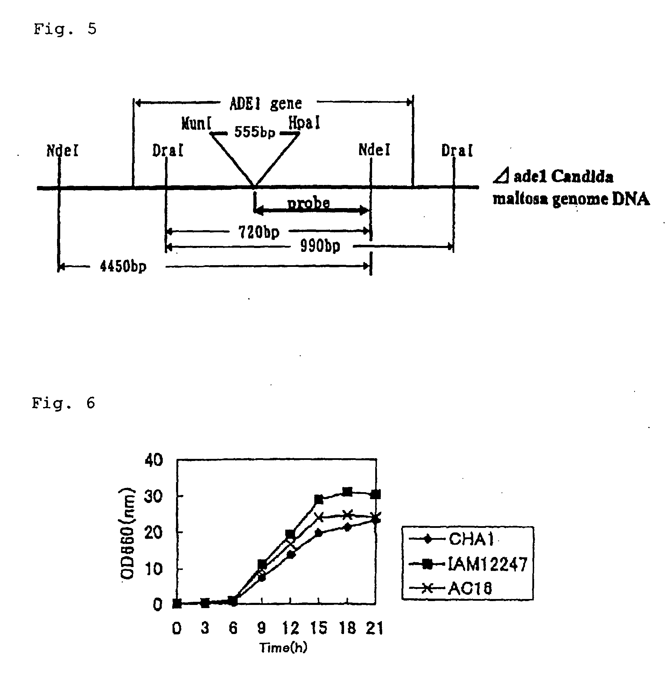

The chromosomal DNAs were extracted using the chromosome extraction kit Gentle-Kun (product of Takara Shuzo). A 5-µg portion of each chromosomal DNA obtained was cleaved by three methods, namely with *Dra*I, *Dra*I + *Nde*I, or *Nde*I, followed by 0.8% agarose gel electrophoresis. According to Molecular cloning: A Laboratory Manual, Second Edition, 9.31-9.57, Cold Spring Harbor Laboratory Press (1989), the bands were transferred to a Hybond N+ filter (product of Amersham) overnight. The detection probe used in southern hybridization was the *Hpa*I-*Nde*I fragment (264 bp), which is a sequence within the ADE1 gene, enzyme-labeled using the GeneImage labeling/detection kit (product of Amersham) (SEQ ID NO:5) . After hybridization, the filter was washed, and DNA band detection was performed using the fluorescence emitting reagent attached to the same kit. Upon comparison of the bands thus detected with that obtained with the wild strain IAM 12247, it was confirmed, in two strains (C16, C17), that shortening by about 550 bp had occurred within the ADE1 gene (Fig. 4), namely the ADE1 gene on the chromosome had been disrupted. In Fig. 5, there was shown a restriction enzyme map of the vicinity of the disrupted ADE1 gene in the ADE1 gene disrupted *Candida maltosa* chromosome as revealed by southern hybridization. "555 bp" indicates the deleted portion between the *Mun*I and *Hpa*I sites. "probe" denotes the partial DNA used in southern hybridization. When this probe was used, the base lengths indicated below the chromosomal DNA were confirmed, as illustrated in Fig. 4. With the wild strain IAM 12247, values resulting from addition of about 550 bp to these values were confirmed.

One of the ADE1 gene disrupted strains thus obtained was named as *Candida maltosa* AC16.

### (Example 6) Examination of the proliferation potency of the ADE1 gene disrupted strain

The proliferation potency of the gene disrupted AC16 strain in complete medium (YPD medium containing adenine (100 mg/L) and histidine (100 mg/L)) was compared with those of the wild strain IAM 12247 and the CHA1 strain. Each yeast was inoculated into 10 ml of the YPD medium and, after overnight culture, the culture was inoculated, in an inoculum size of 1%, into 10 ml of the same medium. OD measurements were made at 660 nm at time intervals. As a result, while the growth of the AC16 strain and of the wild strain arrived at the final stage in 15 hours, the growth of the AC16 strain was only 83% of that of the wild strain. On the other hand, CHA1 grew to a concentration comparable to that of the AC16 strain; the AC16 strain arrived at that concentration earlier by 6 hours, however (Fig. 6). Thus, it was demonstrated that the AC16 strain is superior in proliferation potency in complete medium to the CHA1 strain.

### (Example 7) Examination of the fat/oil utilizing capacity of the ADE1 gene disrupted strain

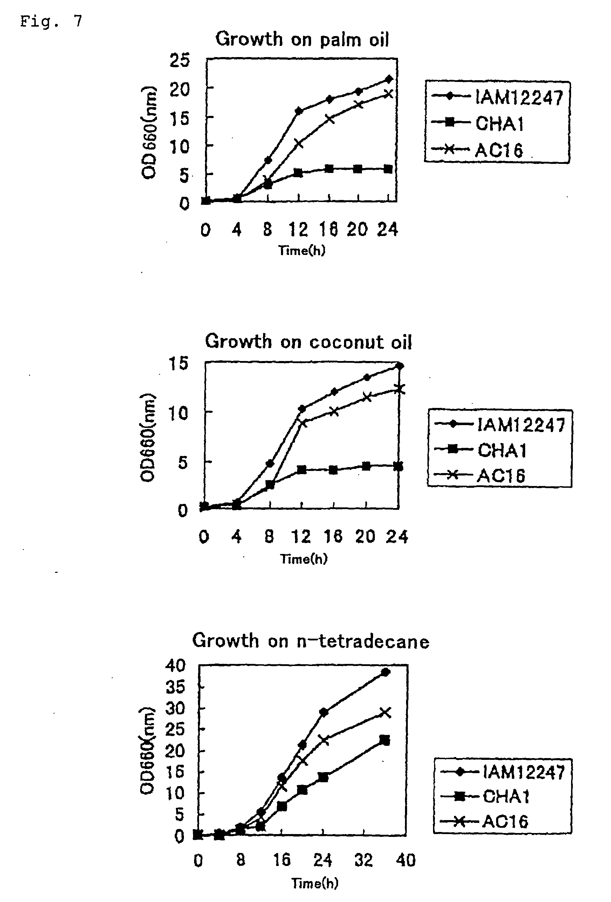

Among the gene disrupted strains, the AC16 strain was compared in oil/fat utilizing capacity with the wild strain IAM 12247 and the CHA1 strain. Used as the fat/oil were palm oil, coconut oil, and tetradecane, which is an n-alkane containing 14 carbon atoms. Each yeast was inoculated into 50 ml of YNB medium supplemented with 2% of an oil (500 ml Sakaguchi flask) and, after overnight culture, the yeast suspension was inoculated, in an inoculum size of 10%, into the same volume of the medium, and OD measurements were made at 660 nm at time intervals. As a result, it was found that while the AC16 strain obtained this time is inferior in proliferation potency by about 15% to the wild strain in palm oil and coconut oil, it is higher in oil/fat utilizing potency by about 4 times (6 times after 32 hours) than the CHA1 strain. In the case of tetradecane, CHA1 showed higher proliferation potency than in fats/oils (palm oil and coconut oil) but the potency was 55% of that of the wild strain and 80% of that of the AC16 strain. In this manner, the ADE1 disrupted strain was superior in oil/fat and alkane utilizing capacity than the CHA1 strain. The growth curves in the respective carbon sources were shown in Fig. 7.

### (Example 8) Synthesis of genes involved in polyester synthesis

The enzyme genes involved in polyester synthesis were designed based on the amino acid sequences of the *Aeromonas caviae*-derived PHA polymerase and the (R) form-specific enoyl-CoA hydratase, which converts enoyl-CoA, which is an intermediate in the β-oxidation pathway, to the monomer (R)-3-hydroxyacyl-CoA (Fukui T., et al., FEMS Microbiology Letters, vol. 170, p69-75 (1999)).

In designing the base sequences, CTG was not assigned to the leucine codon, since *Candida maltosa* is a yeast in which the CTG codon is not translated into leucine but into serine. Those codons most frequently used in *Candida maltosa* were preferentially selected as the codons corresponding to the respective amino acids. As for the codon frequencies, reference was made to Mauersberger S., et al. (authors), *Candida maltosa,* in Wolf K. (ed.), Nonconventional Yeasts in Biotechnology. p524-527. The thus-designed sequences ORF2 and ORF3 were shown under SEQ ID NO:6 and SEQ ID NO:7, respectively. The DNAs were wholly synthesized according to these base sequences.

### (Example 9) Construction of a recombinant plasmid for polyester synthesis

In order that the above ORF2 and ORF3 might be expressed in *Candida maltosa,* it was decided that the promoter ALK1p (SEQ ID NO:8) of the *Candida maltosa* Alk1 gene be ligated to the 5' upstream of each of ORF2 and ORF3 and the terminator ALK1t (SEQ ID NO:9) of the *Candida maltosa* Alk1 gene to the 3' downstream. For preparing restriction enzyme sites for ligating the promoter and terminator to the structural gene, the PCR method was utilized. As for the PCR conditions, a cycle comprising 1 minute at 94°C, 2 minutes at 55°C and 3 minutes at 72°C was repeated 25 times to amplify the desired gene fragment. The polymerase used was Takara Shuzo's ExTaq. As for the promoter portion, ALK1p having a 5'-terminal *Sal*I site and a 3'-terminal *Nde*I site was constructed using the sequence under SEQ ID NO:8 as the template and further using the sequences under SEQ ID NO:10 and SEQ ID NO:11. As for the terminator portion, ALK1t having a 5'-terminal *Hind*III site and a 3'-terminal *Eco*RV site was constructed using the sequence under SEQ ID NO:9 as the template and further using the sequences under SEQ ID NO:12 and SEQ ID NO:13. As the vector to be used finally ligating ORF2 and ORF3 was the vector pUTA1 (Fig. 2, below) derived from pUTU1 (M. Ohkuma, et al., J. Biol. Chem., vol. 273, p3948-3953 (1998)) resulting from ligating of the autonomously replicating sequence (ARS) of *Candida maltosa* (Gen Bank D29758) and the URA3 gene (Gen Bank D12720) to pUC19, and the ADE1 gene of *Candida maltosa,* for conversion of the marker gene from uracil to adenine. pUTA1 was constructed by removing the URA3 gene from pUTU1 using *Xho*I, followed by ligating thereto the ADE1 gene fragment cleaved using *Sal*I.

ALK1p was ligated to pUCNT (described in WO 94/03613) at the *Pvu*II-*Nde*I site thereof, and ALK1t to pUCNT at the *Hind*III-*Ssp*I site to construct pUAL1 Then, a plasmid, pUAL-ORF2, was constructed by ligating ORF2 to pUAL1 at the *Nde*I-*Pst*I site thereof. Further, pUAL-ORF3 was constructed by ligating ORF3 to pUCNT-ALK1t, constructed in the course of pUAL1 construction, at the *Nde*I-*Hind*III site thereof, followed further ligating of ALK1p

Then, ORF2 was cleaved, together with the upstream promoter and downstream terminator, from the plasmid pUAL-ORF2 using *Eco*T22I and ligated to pUTA1 at the *Pst*I site thereof to construct pUTA-ORF2. Further, ORF3 was cleaved, together with the upstream promoter and downstream terminator, from pUAL-ORF3 using *Sal*I and ligated to pUTA-ORF2 at the *Sal*I site thereof to construct the plasmid pUTA-ORF23 (Fig. 2, above). In the above manner, a gene expression cassette for polyester production in *Candida maltosa* was constructed.

### (Example 10) Production of a transformant for polyester production

For transformation to the AC16 strain and CHA1 strain, each yeast was cultured in YM medium until the OD at 660 nm arrived at 0.8 to 1.0, cells were recovered by centrifugation, and the above expression vector (pUTA-ORF23) was transformed into these cells. In gene transformation, the process from preparation of competent cells to gene transformation was performed using GENO TECH's FastTrack^{TM}-Yeast Transformation_{SM} Kit. An M medium plate was smeared with the cells after gene transformation, and cultured for 2 days. Since the ADE1 gene has been inserted in pUTA-ORF23, adenine synthesis is possible. Therefore, cells grown in an adenine-free medium are transformants. Cells grown in an adenine-free medium were recovered to give a transformant strain, AC16 (ORF23). In the same manner, a transformant strain, CHA1 (ORF23) was obtained from transformation of PUTA-ORF23 into the CHA1 strain. Further, the AC16 strain and CHA1 strain were transformed in the same manner using the polyester synthase-free control vector pUTA1 to give transformant strains, AC16 (CT) and CHA1 (ORFCT).

### (Example 11) Polyester production culture

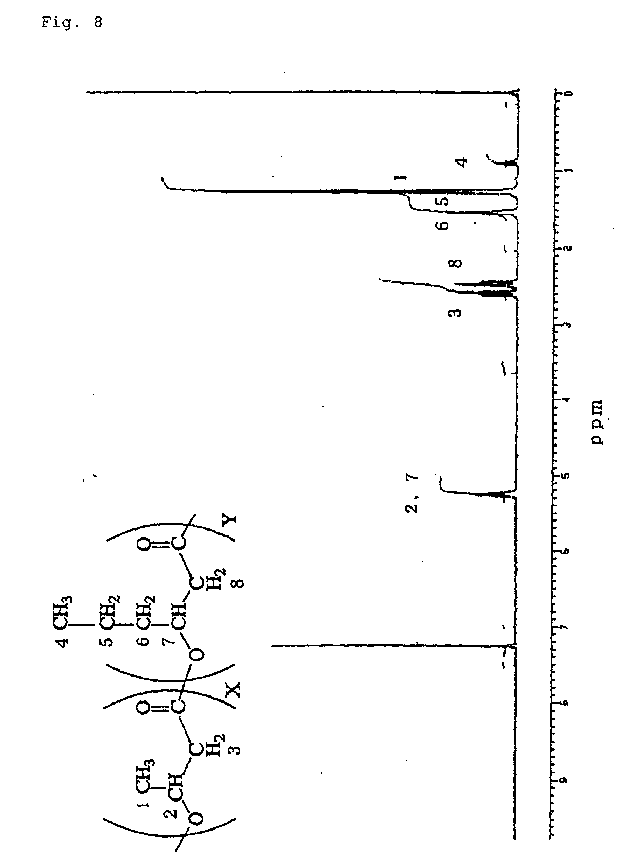

All polyester production cultures were carried out using 2-L Sakaguchi flasks. First, 100 µl of a glycerol stock containing each recombinant strain was inoculated into 10 ml of SD medium and cultured overnight. The thus-cultured yeast 1 ml was inoculated into 50 ml of ammonium sulfate-containing M medium for glucose starvation (containing 100 g/L of ammonium sulfate). Culture was carried out in this medium for 2 days for complete exhaustion of glucose in the medium. Then, 10 ml of the yeast was inoculated into 50 ml of polyester production medium (500-ml Sakaguchi flask) and cultured for 8 hours and, then, the whole culture was transferred to 500 ml of the same polyester production medium, and production culture was performed for 120 hours. The culture medium was autoclaved, and cells were recovered by centrifugation, washed with methanol and then lyophilized. The dried cells obtained were ground, 100 ml of chloroform was added thereto, and the mixture was stirred overnight for polyester extraction. The chloroform solution was recovered by filtration and concentrated to 1 to 2 ml using an evaporator, and 10 ml of hexane was added to the concentrate to cause precipitation of a hexane-insoluble matter. The AC16 (ORF23) strain gave a precipitate, whereas the CHA1 (ORF23) gave only a trace amount of precipitate. The AC16 (CT) strain gave no precipitate at all. The hexane precipitate obtained from the AC16 (ORF23) strain was dried and then subjected to 400 MHz proton NMR analysis (JEOL, JNM-EX400). The NMR chart obtained was shown in Fig. 8. The correspondence of each peak to each proton of the polyester P(3HB-co-3HH) was indicated by a number. Analysis revealed that the 3HH fraction was 9.7%. The yield of the polyester was 0.1% based on the dry yeast weight. In the CHA1 (ORF23) strain, the polyester could be also identified but the yield was at most 0.05%. It was thus revealed that the copolyester P(3HB-co-3HH) can be produced using the ADE1 gene disrupted strain of *Candida maltosa* according to the invention. Furthermore, it was demonstrated that this disrupted strain transformant is superior in polyester productivity to the ADE1 gene-mutated yeast CHA1 obtained by mutagenesis treatment. Thus, the utility of the ADE1 gene disrupted yeast of the present invention has been established.

### INDUSTRIAL APPLICABILITY

The present invention has demonstrated the utility of ADE1 gene disrupted yeast strains. ADE1 gene disrupted strains of *Candida maltosa* can be expected to be usable as host yeasts for gene recombination in the expression of genes or in the production of gene expression products. It is also possible to impart them with further different auxotrophy, and this leads to the development of yeasts which can be used safely as recombinants.

## Claims

1. A yeast
in which ADE1 gene in chromosomal DNA has been disrupted by homologous recombination with an ADE1 DNA fragment.

2. The ADE1 gene disrupted yeast according to Claim 1,
which belongs to any of the genera *Aciculoconidium, Ambrosiozyma, Arthroascus, Arxiozyma, Ashbya, Babjevia, Bensingtonia, Botryoascus, Botryozyma, Brettanomyces, Bullera, Bulleromyces, Candida, Citeromyces, Clavispora, Cryptococcus, Cystofilobasidium, Debaryomyces, Dekkara, Dipodascopsis, Dipodascus, Eeniella, Endomycopsella, Eremascus, Eremothecium, Erythrobasidium, Fellomyces, Filobasidium, Galactomyces, Geotrichum, Guilliermondella, Hanseniaspora, Hansenula, Hasegawaea, Holtermannia, Hormoascus, Hyphopichia, Issatchenkia, Kloeckera, Kloeckeraspora, Kluyveromyces, Kondoa, Kuraishia, Kurtzmanomyces, Leucosporidium, Lipomyces, Lodderomyces, Malassezia, Metschnikowia, Mrakia, Myxozyma, Nadsonia, Nakazawaea, Nematospora, Ogataea, Oosporldlum, Pachysolen, Phachytichospora, Phaffia, Pichia, Rhodosporidium, Rhodotorula, Saccharomyces, Saccharomycodes, Saccharomycopsis, Saitoella, Sakaguchia, Saturnospora, Schizoblastosporion, Schizosaccharomyces, Schwanniomyces, Sporidiobolus, Sporobolomyces, Sporopachydermia, Stephanoascus, Sterigmatomyces, Sterigmatosporidium, Symbiotaphrina, Sympodiomyces, Sympodiomycopsis, Torulaspora, Trichosporiella, Trichosporon, Trigonopsis, Tsuchiyaea, Udeniomyces, Waltomyces, Wickerhamia, Wickerhamiella, Williopsis, Yamadazyma, Yarrowia, Zygoascus, Zygosaccharomyces, Zygowilliopsis,* and *Zygozyma.*

3. The ADE1 gene disrupted yeast according to Claim 1,
which belongs to any of the genera *Candida, Yarrowia, Kluyveromyces,* and *Hanseniaspora.*

4. The ADE1 gene disrupted yeast according to Claim 1, which belongs to the genus *Candida.*

5. The ADE1 gene disrupted yeast according to Claim 4, which is *Candida maltosa.*

6. The ADE1 gene disrupted yeast according to Claim 5, which is *Candida maltosa* AC16 (FERM BP-7366).

7. An ADE1 gene disrupted yeast transformant according to any one of Claims 1 to 6,
which is transformed with a DNA sequence containing a homologous or heterologous gene.

8. The transformant according to Claim 7,
wherein the ADE1 gene disrupted yeast belongs to the genus *Candida.*

9. The transformant according to Claim 8,
wherein the ADE1 gene disrupted yeast is *Candida maltosa.*

10. The transformant according to Claim 9,
wherein the ADE1 gene disrupted yeast is *Candida maltosa* AC16 (FERM BP-7366).

11. A method of producing gene expression products
which comprises cultivating the transformant according to any one of Claims 7 to 10 and recovering a homologous or heterologous gene expression product from the culture obtained.

12. The method of producing gene expression products according to Claim 11,
wherein the gene expression product is a polyester.


SEQUENCE LISTING

<110> KANEKA CORPORATION

<120> GENE DISRUPTED YEAST

<130> T648.PBT-3

<150> JP2001-011191
<151> 2001-01-19

<160> 13

<210> 1
<211> 1820
<212> DNA
<213> Candida maltosa

<220>
<221> CDS
<222> 538..1413
<223> Ade1

<400> 1
gatccccttc ttcaaacctt taaatgacat tgtttcgttt ctctatgttt ggtatcggtt 60
cttcttcttc ttcaaaaaaa aggggggcac tattcaaaaa aaaatattat aacagtatga 120
tttttttccc tctcccgtcg attgaggttt tttttttctc tttcgtcttg gtcttttgct 180
tttcactcca aaaatggaaa cacgcgcggc tcaactcgaa atccgtgatc aaaaaaataa 240
aggctgtgag tttcgagcca ataattatga attagtggta ttttttttaa agataaataa 300

tcaagaatcg cattagggag acgaatatgc gttattcaaa taaaaagaca attcttttag 360
ggtagcattt cccttcaagt tcatcccaca tgtacattaa tgtcaatgat gtcgcagaag 420
ttaaattagc agaagaaaaa aaaaatgtga attactccga gtcaactctt ctttctcttc 480
ttctttttct tctttatcac cataatcacc accaccacca ccaccaccag ctcccagatg 540
acttcaacta acttagaagg aactttccca ttgattgcca aaggtaaagt cagagatatt 600
taccaagttg acgacaacac tcttttattc gttgctactg atagaatttc cgcatacgat 660
gtgattatgt ctaatggtat cccaaataaa ggtaaaatct taaccaaatt gtctgaattc 720
tggtttgatt tcttgccaat tgaaaaccat ttaatcaaag gagacatttt ccaaaaatat 780
cctcaactag aaccatatag aaaccaattg gaaggcagat ccttacttgt tagaaaattg 840
aaattgatcc ctcttgaagt tattgttaga ggttacatca ccggttccgg ctggaaagaa 900
taccaaaaat ctaaaaccgt ccacggtatt cctattggtg atgtggttga atcacaacaa 960
atcactccta tcttcacccc atccactaaa gcagaacaag gtgaacatga tgaaaatatc 1020
accaaagaac aagctgacaa gattgttgga aaagaattat gtgatagaat tgaaaaaatt 1080
gctattgatt tgtacaccaa agccagagat tacgctgcca ctaaaggaat tattatcgct 1140
gatactaaat ttgaatttgg tttagatggt gacaacatcg ttcttgttga cgaagtttta 1200
actccagatt cttccagatt ctggaatgct gctaaatacg aagttggtaa atctcaagac 1260
tcttacgata aacaattttt gagagattgg ttaacttcta atggtgttgc tggtaaagat 1320
ggtgttgcta tgcctgaaga cattgtcact gaaaccaaga gcaaatacgt tgaagcttac 1380
gaaaatttaa ctggtgacaa atggcaagaa taaattaagg atatctatta ttaaagcttt 1440
ctatttatcc caaactttcg tagtattttc tgacatgttc agatgttttt actttatctt 1500
tcctgaaatt tttgatttct aaccgactct tgcatgtagc tcttgataat gcaacatatg 1560
cttgaccatt agcaaaactt ctacctaaat ctattttgac tctgtccaaa gtttgacctt 1620
gagctttgtg gatcgacatc gcccacgaca agatcatttg gtttgttttt atggtgggtt 1680
attggcactt ggtgcaactg atggtttaac tttggaagag gctaagaaat tgaagacttg 1740
gaatgaagaa cgtgcatctg atttcaaatt gggtgaagaa ttgacttata cttgttataa 1800
aatgtatcat gatgttgatc 1820

<210> 2
<211> 1032

<212> DNA

<213> Candida maltosa

<400> 2

attataacag tatgattttt ttccctctcc cgtcgattga ggtttttttt ttctctttcg 60

tcttggtctt ttgcttttca ctccaaaaat ggaaacacgc gcggctcaac tcgaaatccg 120

tgatcaaaaa aataaaggct gtgagtttcg agccaataat tatgaattag tggtattttt 180

tttaaagata aataatcaag aatcgcatta gggagacgaa tatgcgttat tcaaataaaa 240

agacaattct tttagggtag catttccctt caagttcatc ccacatgtac attaatgtca 300

atgatgtcgc agaagttaaa ttagcagaag aaaaaaaaaa tgtgaattac tccgagtcaa 360

ctcttctttc tcttcttctt tttcttcttt atcaccataa tcaccaccac caccaccacc 420

accagctccc agatgacttc aactaactta gaaggaactt tcccattgat tgccaaaggt 480

aaagtcagag atatttacca agttgacgac aacactcttt tattcgttgc tactgataga 540

atttccgcat acgatgtgat tatgtctaat ggtatcccaa ataaaggtaa aatcttaacc 600

aaattgtctg aattctggtt tgatttcttg ccaattaact tctaatggtg ttgctggtaa 660

agatggtgtt gctatgcctg aagacattgt cactgaaacc aagagcaaat acgttgaagc 720

ttacgaaaat ttaactggtg acaaatggca agaataaatt aaggatatct attattaaag 780

ctttctattt atcccaaact ttcgtagtat tttctgacat gttcagatgt ttttacttta 840

tctttcctga aatttttgat ttctaaccga ctcttgcatg tagctcttga taatgcaaca 900

tatgcttgac cattagcaaa acttctacct aaatctattt tgactctgtc caaagtttga 960

ccttgagctt tgtggatcga catcgcccac gacaagatca tttggtttgt ttttatggtg 1020

ggttattggc ac 1032

<210> 3

<211> 24

<212> DNA

<213> Artificial Sequence

<220>

<223> primer

<400> 3
   taacagtatg atttttttcc ctct 24

<210> 4
<211> 24
<212> DNA
<213> Artificial Sequence

<220>
<223> primer

<400> 4
aacaaaccaa atgatcttgt cgtg 24

<210> 5
<211> 264
<212> DNA
<213> Candida maltosa

<220>
<221> CDS
<223> probe

<400> 5
aacttctaat ggtgttgctg gtaaagatgg tgttgctatg cctgaagaca ttgtcactga 60
aaccaagagc aaatacgttg aagcttacga aaatttaact ggtgacaaat ggcaagaata 120
aattaaggat atctattatt aaagctttct atttatccca aactttcgta gtattttctg 180

acatgttcag atgtttttac tttatctttc ctgaaatttt tgatttctaa ccgactcttg 240

catgtagctc ttgataatgc aaca 264

<210> 6

<211> 1785

<212> DNA

<213> Artificial Sequence

<220>

<221> CDS

<222> 1..1785

<223> ORF2

<400> 6

atg tct caa cca tct tat ggt cca ttg ttc gaa gct ttg gct cat tac 48

aat gat aaa ttg ttg gct atg gct aaa gct caa acc gaa aga act gct 96

caa gcc ttg ttg caa act aac ttg gat gat ttg ggt caa gtt ttg gaa 144

caa ggt tct caa caa cca tgg caa ttg att caa gct caa atg aat tgg 192

tgg caa gat caa tta aaa ttg atg caa cac act ttg tta aaa tct gct 240

ggt caa cca tct gaa cca gtt att act cca gaa aga tct gat aga aga 288

ttt aaa gct gaa gct tgg tct gaa caa cca att tat gat tac tta aaa 336

caa tcc tat ttg tta act gct aga cat ttg ttg gct tct gtt gat gct 384

ttg gaa ggt gtc cca caa aaa tct aga gaa aga ttg aga ttc ttt act 432

aga caa tac gtc aac gct atg gct cca tct aat ttc ttg gct act aac 480

cca gaa ttg tta aaa ttg act ttg gaa tcc gat ggt caa aat ttg gtt 528

aga ggt ttg gct tta ttg gct gaa gat ttg gaa aga tct gct gat caa 576

tta aac att aga ttg act gat gaa tcc gct ttt gaa tta ggt aga gat 624

ttg gct ttg act cca ggt aga gtt gtt caa aga act gaa tta tat gaa 672

tta att caa tac tct cca act act gaa acc gtt ggt aaa acc cca gtt 720

ttg atc gtt cca cca ttc att aat aaa tat tac att atg gat atg aga 768

cca caa aac tcc ttg gtc gct tgg ttg gtc gct caa ggt caa acc gtt 816

ttc atg att tcc tgg aga aac cca ggt gtt gct caa gct caa att gat 864

tta gat gat tat gtt gtt gat ggt gtc att gct gct ttg gat ggt gtt 912

gaa gcc gct act ggt gaa aga gaa gtt cac ggt att ggt tac tgt att 960

ggt ggt acc gct ttg tct tta gct atg ggt tgg ttg gcc gcc aga aga 1008

caa aaa caa aga gtt aga act gct act ttg ttt act act ttg ttg gat 1056

ttc tcc caa cca ggt gaa ttg ggt att ttt att cat gaa cca att atc 1104

gcc gcc tta gaa gcc caa aat gaa gct aaa ggt att atg gat ggt aga 1152

caa ttg gcc gtc tcc ttc tct ttg ttg aga gaa aac tct tta tat tgg 1200

aat tac tat att gat tct tac tta aaa ggt caa tct cca gtt gct ttt 1248

gat ttg ttg cac tgg aac tct gat tct act aat gtt gcc ggt aaa act 1296

cat aac tct ttg ttg aga aga tta tat ttg gaa aat caa ttg gtt aaa 1344

ggt gaa tta aaa att aga aac act aga att gat tta ggt aaa gtt aaa 1392

act cca gtt ttg ttg gtt tct gcc gtt gat gat cac att gct tta tgg 1440

caa ggt acc tgg caa ggt atg aaa ttg ttc ggt ggt gaa caa aga ttt 1488

tta ttg gcc gaa tcc ggt cat att gct ggt att att aat cca cca gct 1536

gct aac aaa tac ggt ttc tgg cac aat ggt gct gaa gct gaa tct cca 1584

gaa tct tgg ttg gct ggt gcc acc cat caa ggt ggt tcc tgg tgg cca 1632

gaa atg atg ggt ttt att caa aac aga gat gaa ggt tct gaa cca gtc 1680

cca gcc aga gtc cca gaa gaa ggt ttg gct cca gct cca ggt cac tat 1728

gtc aaa gtt aga tta aac cca gtt ttc gct tgt cca acc gaa gaa gat 1776

gct gct taa 1785

<210> 7

<211> 405

<212> DNA

<213>Artificial Sequence

<220>

<221> CDS

<222> 1..404

<223> ORF3

<400> 7

atg tct gct caa tcc ttg gaa gtt ggt caa aaa gct aga tta tct aaa 48

aga ttc ggt gcc gcc gaa gtt gct gct ttt gct gcc tta tct gaa gat 96

ttc aac cca ttg cac ttg gat cca gct ttt gct gct act acc gcc ttc 144

gaa aga cca atc gtc cat ggt atg ttg tta gct tct tta ttt tcc ggt 192

ttg ttg ggt caa caa ttg cca ggt aaa ggt tct att tat ttg ggt caa 240

tct tta tct ttc aaa ttg cca gtc ttt gtc ggt gat gaa gtt acc gct 288

gaa gtt gaa gtt act gct ttg aga gaa gat aaa cca att gct act ttg 336

act act aga att ttc act caa ggt ggt gct tta gct gtt acc ggt gaa 384

gct gtt gtc aaa ttg cca taa 405

<210> 8

<211> 1017

<212> DNA

<213> Candida maltosa

<220>

<223> promoter ALK1p

<400> 8

atgcatgaac aggatttaat cccaagaaaa aagtctattt tctattttca caaggaaact 60

ggaaaaacct ttttgtgttt tgaagtagct ccgtaataac ctgtaaaaaa ataaattttg 120

aagatttgac ttgctgatga aaatgctatc agtgtagctc tagacttgat actagactat 180

gatggcaaca catggtggtc aacgtgcaag acatcaccca atgagaagac tgctaaccag 240

aaaaaaaagg ggacaaaaga aaaactcgag agaaaaagtc aaattggtgt aaaattggct 300
atttttggta ctttcctaat ggggaaatta attgtttaaa attccagttt ttccagagtt 360
aagatttcga ccaattattt ttaatccata tgatcttcat cattatcaac ttgtgaaaaa 420
taataatcga ggtacgttta atacgagata ttagtctacg gctatgaatg ttggatatac 480
ttcattgacg atcagaagct tgattggtta ttcaggtgca tgtgtggata taaacccaac 540
aaattatcta gcaactgtgc cttccccaca ttggtcaaag aaaccctaaa gcaaattaaa 600
atctggataa ataaatcatt catttcacat tttccggtta gtataaggtt ttttaaattt 660
tttttacag tttagccctt tcaattacca aatacggtaa caatgtgctt tgtaacatgc 720
aggggatttt ctccgttgct gttttctcca catgctttta atgtgtaata aattaaaaaa 780
attacaaaga aaaaccggca tataagcatc ggagtttaca ttgttaacta actgcaaaat 840
ggcgatgttt caaatcaaca aaatttaaaa aaaccccaaa aaaaaagtat catataaatt 900
aaactcaaaa tcctttgat tgcataaaat ttttaaatct cttctttttt ttcttttta 960
ctttcttatc tattctattc tttttttata tatctaattc atttataaca tctggtc 1017

<210> 9
<211> 218
<212> DNA
<213> Candida maltosa

<220>
<223> terminater ALK1t

<400> 9
atagatggat ttttcttttt tatgtgtatt tccggttaat aaatgtttaa attttttttt 60
taataaaaat atttgtagtt atttatatgc aaaaaaaaaa aatattcaaa gcaatcttcc 120
tttctttctt tatctttccc ccatgctaag gtctaaaaca ccacaactta aaacccaact 180
taaccgtata atactaagat caatctccaa agatgcat 218

<210> 10

<211> 46

<212> DNA

<213> Artificial Sequence

<220>

<223> primer

<400> 10

tttttcagct ggagctcgtc gacatgcatg aacaggattt aatccc 46

<210> 11

<211> 39

<212> DNA

<213> Artificial Sequence

<220>

<223>primer

<400> 11

ccggaattcc atatgcagat gttataaatg aattagata 39

<210> 12

<211> 32

<212> DNA

<213> Artificial Sequence

<220>

<223> primer

<400> 12

cggaagctta tagatggatt tttcttttt at 32

<210> 13

<211> 45

<212> DNA

<213> Artificial Sequence

<220>

<223> primer

<400> 13

tttttgatatc gagctcgtcg acatgcatct ttggagattg atctt 45